# EUROPÄISCHE PATENTSCHRIFT

(11) **EP 0 362 616 B1**
(45) Veröffentlichungstag und Bekanntmachung des Hinweises auf die Patenterteilung: **02.02.1994**
(21) Anmeldenummer: 89117280.1
(22) Anmeldetag: 19.09.1989
(51) Int. Cl.: A61B 5/103

(54) **Vorrichtung und Verfahren zur Messung der elastischen und viskoelastischen Verformbarkeit der Haut**
Apparatus and process for measuring the elastic and viscoelastic deformation of the skin
Appareil et procédé pour mesurer la déformation élastique et viscoélastique de la peau

(30) Priorität: 26.09.1988 DE 3832690
(43) Veröffentlichungstag der Anmeldung: 11.04.1990
(73) Patentinhaber: COURAGE + KHAZAKA ELECTRONIC GmbH, D-50829 Köln (DE)
(72) Erfinder: Courage, Wilfried, Dipl.-Ing., D-5000 Köln 41 (DE)
(74) Vertreter: Dallmeyer, Georg, Dipl.-Ing.

(56) Entgegenhaltungen:
- DE-A- 3 304 503
- DE-U- 8 701 560
- DE-U- 8 703 658
- GB-A- 2 173 896

## Beschreibung

Die Erfindung betrifft eine Vorrichtung zur Messung der elastischen und viskoelastischen Verformbarkeit von Haut mit einer Meßsonde, die eine Wegmeßeinrichtung zur Messung des Deformationsweges der Hautoberfläche aufweist, und mit einer elektronischen Auswerteeinrichtung sowie ein Verfahren zur Messung der elastischen und plastischen Verformbarkeit von Haut durch Beaufschlagung der Haut mit einem Druck über eine Meßsonde und durch Messen der Hautdeformation auf Grund der Druckbelastung über eine Deformationswegmessung.

In der Dermatologie und der Kosmetik ist es häufig notwendig, Messungen der Hautelastizität durchzuführen, umd den Erfolg von Therapien oder den Erfolg kosmetischer Mittel überprüfen zu können.

Aus der DE-PS 29 09 092 ist eine Vorrichtung zur Messung der Elastizität der menschlichen Haut mit einem mit vorbestimmbaren Druck auf die Hautoberfläche andrückbaren Taster bekannt. Bei dieser Vorrichtung wird der Taster durch ein vorwählbares Gewicht beaufschlagt und der Einfederungsweg des Tasters in die zu prüfende Hautoberfläche mit Hilfe einer Wegmeßeinrichtung festgestellt. Alternativ ist es möglich, den Taster an seiner Spitze mit einer Saugglocke zu versehen, die mit Unterdruck beaufschlagt wird, um mit Hilfe des Tasters nicht nur eine Druckbelastungsprüfung sondern auch eine Zugprüfung durchführen zu können. In diesem Fall saugt sich der Taster an der Hautoberfläche fest und wird anschließend mit einem Gewicht über eine Umlenkrolle derart belastet, daß er auf die Hautoberfläche eine Zugkraft ausübt. Dabei wird der Verschiebungsweg des Tasters in Abhängigkeit von der Zugkraft mit Hilfe einer elektronischen Auswerteeinrichtung gemessen. Eine derartige Meßvorrichtung ist mechanisch aufwendig, wobei die Messung selbst auf Grund der geringen Gewichte schwierig durchzuführen ist, da für die Messung die zu untersuchende Hautoberfläche absolut stilliegen muß. Außerdem ist mit der beschriebenen Vorrichtung keine zufriedenstellende Genauigkeit erzielbar, da bei den geringen Drücken und den daraus resultierenden geringen Auflagegewichten Störfaktoren wie die Seilreibung und die Lagerreibung der Seilrolle sowie Bewegungen der Hautoberfläche in die Messung eingehen. Schließlich ist es wegen der Gewichte erforderlich, daß die zu untersuchende Hautoberfläche horizontal liegt.

Es ist ferner eine Vorrichtung zur Messung der menschlichen Hautelastizität aus der EP 0 255 809 bekannt, bei der eine Unterdruckglocke auf die zu untersuchende Hautoberfläche aufgesetzt wird, die über eine Leitung mit einem Unterdruckmeßgerät und einer Meßkammer verbunden ist, in der ein Kolben beweglich angeordnet ist. Zur Beaufschlagung der Saugglocke mit einem Unterdruck wird der Kolben der Meßkammer aus einer definierten Null-Lage bis zu einem vorbestimmten Unterdruck zurückbewegt und an der Meßkammer das hierfür benötigte Absaugvolumen festgestellt, das von dem sich in die Saugglocke hineinwölbenden Hautvolumen abhängt. Diese Vorrichtung hat den Nachteil, daß für die Volumenmessung eine relativ große Hautfläche benötigt wird und folglich die Messung kleinerer Hautflächen oder unterschiedlicher Hautschichten z.B. der obersten Hautschicht, die für die Kosmetik von besonderer Bedeutung ist, nicht möglich ist.

Aus der DE-OS 36 12 312 ist eine Vorrichtung zur Untersuchung der Elastizität der Hautoberfläche bekannt, bei der die Verformung der Haut durch Anblasen mit einem Gasstrom erzeugt wird. Der Grad der Verformung wird über die Reflektion von Licht an der verformten Stelle ermittelt. Eine Lichtschranke ist nicht vorhanden.

Aus der FR-OS 26 03 183 ist eine Meßvorrichtung zur Messung der Hautcharakteristiken bekannt, bei der ein Teil mit Federkraft in die Hautoberfläche gedrückt wird, das in Abhängigkeit von der Verformung der Haut unterschiedlich tief eintaucht. Das der Haut gegenüberliegende Ende dieses Teils beeinflußt in Abhängigkeit von der Eindringtiefe in die Haut den Lichtweg einer Lichtschranke.

Es ist ferner ein optischer Sensor bekannt (DE 87 03 658) bei dem Sender und Empfänger in einem U-förmigen Gehäuse untergebracht sind, wobei der Sender in dem einen Schenkel und der Empfänger in dem anderen Schenkel des U-förmigen Gehäuses angeordnet sind und der Strahlengang senkrecht zwischen den Schenkeln verläuft.

Der Erfindung liegt die Aufgabe zugrunde, eine Vorrichtung und ein Verfahren zur Messung der elastischen und viskoelastischen Verformbarkeit von Haut zu schaffen, das eine verbesserte Meßgenauigkeit und eine hohe Reproduzierbarkeit der Meßwerte ermöglicht, das auch die Messung der Flexibilität oberster Hautschichten zuläßt und das nicht stoßempfindlich ist.

Zur Lösung dieser Aufgabe ist erfindungsgemäß vorgesehen,
- daß die Meßsonde einen die Haut mit einem pneumatischen Druck beaufschlagenden Meßsondenkanal aufweist,
- daß zur Wegmessung im Bereich des Meßsondenkanals eine Lichtschranke angeordnet ist, deren Meßstrecke quer zum Meßsondenkanal bis zur Ebene seiner Austrittsöffnung verläuft.

Zur Lösung der Aufgabe ist hinsichtlich des Verfahrens erfindungsgemäß vorgesehen,
- daß der Druck pneumatisch auf die zu messende Hautoberfläche über einen Meßsondenkanal aufgebracht wird,
- daß die Hautdeformation berührungslos mit Hilfe einer in dem druckbeaufschlagten Meßsondenkanal angeordneten Lichtschranke gemessen wird, deren Änderung der Lichtintensität als Maß für die Deformation der Hautoberfläche verwendet wird.

Die Anordnung einer Lichtschranke im Bereich der Austrittsöffnung eines druckbeaufschlagten Meßsondenkanals ermöglicht die unmittelbare unverfälschte Messung der Hautdeformation, wobei gleichzeitig durch die Auflagefläche des Meßkopfes eine definierte Lage für die Ausgangsposition der Hautoberfläche ohne Druckbeaufschlagung gegeben ist. Eine derartige Meßsonde ist unempfindlich gegen Stöße, läßt sich in jeder Position einsetzen und ermöglicht genaue und in hohem Maße reproduzierbare Meßwerte. So sind beispielsweise Deformationen in der Größenordnung von 10 Mikrometern meßbar. Geringfügige Bewegungen der zu untersuchenden Hautoberfläche stören nicht den Meßvorgang.

Vorzugsweise ist vorgesehen, daß der Meßkopf der Meßsonde zumindest teilweise aus einem Glaskörper besteht, durch den die Meßstrecke verläuft. Ein Meßkopf aus Glas ermöglicht, daß die Meßstrecke bis unmittelbar zur Kontaktfläche zwischen Haut und Meßkopf reicht, wodurch selbst geringste Hautdeformationen meßbar sind.

Der Lichtsender und der Lichtempfänger der Lichtschranke können auf gegenüberliegenden Seiten der Meßsonde angeordnet sein und die Meßstrecke kann über Spiegel zweifach umgelenkt sein. Die zweifache Umlenkung der Meßstrecke und die in die Meßsonde integrierten Lichtsender und Lichtempfänger ermöglichen eine kompakte Bauweise der Meßsonde und ermöglichen damit eine freie Beweglichkeit der Meßsonde.

Bei einer Weiterbildung der Erfindung ist vorgesehen, daß die Meßsonde eine elektronische Schaltung zur Steuerung des Lichtsenders und zur Verstärkung des Meßsignals des Lichtempfängers enthält. Die Signalverarbeitung direkt in der Meßsonde vermeidet Übertragungsstörungen oder Fehler.

Vorzugsweise ist vorgesehen, daß der Lichtsender aus einer Infrarotleuchtdiode und der Lichtempfänger aus einer Infrarotphotodiode besteht. Die Verwendung von Infrarotlicht verhindert die Störung der Messung durch das Tageslicht. Zusätzlich kann das Infrarotlicht moduliert sein, um Meßfehler durch Infrarot-Fremdlicht auszuschließen.

Die Austrittsöffnung des Meßsondenkanals kann einen Durchmesser zwischen 2 und 8 mm aufweisen. Bei einem kleinen Austrittsdurchmesser des Meßsondenkanals ist die Messung der Flexibilität oberster Hautschichten möglich. Dagegen kann mit einer größeren Austrittsöffnung auch die Flexibilität tieferer Hautschichten untersucht werden.

Eine Weiterbildung der Erfindung sieht vor, daß der Meßkopf der Meßsonde auswechselbar ist. Die Auswechselbarkeit des Meßkopfes ermöglicht, unterschiedliche Meßsondenkanalquerschnitte oder unterschiedliche Formen der Austrittsöffnung zu verwenden, ohne für jede Variante des Meßsondenkanals eine eigene Meßsonde zu benötigen.

Die Meßsonde kann mit einem von einer Feder vorgespannten Meßkopf vorgesehen sein. Ein derart vorgespannter Meßkopf ermöglicht einen gleichbleibenden und reproduzierbaren Anpreßdruck der Meßsonde gegen die Hautoberfläche.

Vorzugsweise ist vorgesehen, daß eine pneumatische Druckeinrichtung, bestehend aus einer Pumpe, einem Druckspeicher und einem Drosselventil den Druck im Meßsondenkanal erzeugt, wobei die Steuerung der Pumpe des Druckspeicherdruckes und der Drosselventilstellung über einen Mikroprozessor erfolgt, der auch die elektronische Schaltung der Meßsonde ansteuert. Der Mikroprozessor ermöglicht einen vollautomatischen Ablauf einzelner und mehrerer direkt aufeinanderfolgender Messungen mit unterschiedlichen Meßdrücken und Meßprogrammen.

Bei einem bevorzugten Ausführungsbeispiel ist vorgesehen, daß der Druck in dem Meßsondenkanal ein Unterdruck ist. In diesem Fall wird die Hautoberfläche in den Meßsondenkanal durch den Unterdruck hineingezogen, so daß eine berührungslose unmittelbare Messung der Hautdeformation möglich ist.

Bei einem anderen Ausführungsbeispiel ist vorgesehen, daß der Druck in dem Meßsondenkanal ein Überdruck ist, und daß ein in dem Meßsondenkanal frei bewegliches, mit dem Überdruck beaufschlagtes Kolbenelement mit seinem einen Ende die Hautoberfläche berührt und mit seinem anderen Ende in die Meßstrecke der Lichtschranke hineinragt. Durch einfaches Auswechseln eines Meßkopfes und durch entsprechende Ansteuerung der Druckeinrichtung ist es also möglich, alternativ Unterdruckmessungen oder auch Messungen der Eindellung der Hautoberfläche vorzunehmen.

Die Meßsonde kann bei bereits anliegenden Unter- bzw. Überdruck auf die Hautoberfläche aufgesetzt werden oder der Druck in dem Meßsondenkanal kann nach dem Aufsetzen der Meßsonde von Null auf einen vorbestimmten Sollwert erhöht werden. Außerdem ist es möglich, nach dem Aufsetzen der Meßsonde eine Wechselbelastung auf die Hautoberfläche auszuüben, indem der Druck zwischen zwei Grenzwerten mehrfach wechselt. Auch ist es möglich, die Druckbeaufschlagung plötzlich auf den Umgebungsdruck abfallen zu lassen, oder erst nach einer vorgegebenen Zeitspanne. Die erfindungsgemäße Vorrichtung und das erfindungsgemäße Verfahren ermöglichen somit eine Vielfältigkeit von Untersuchungsmethoden, die ein Höchstmaß an Informationen über das elastische und viskoelastische Verhalten der Haut Aufschluß geben können. Eine wichtige Information ist dabei beispielsweise auch nach der Druckbeaufschlagung der Hautfläche die zeitlich abhängige Restdeformation nach Wegfall der Druckbelastung, die ein Maß für die Plastizität der Haut ist.

Im folgenden werden unter Bezugnahme auf die Zeichnungen Ausführungsbeispiele der Erfindung näher erläutert. Es zeigen:
- Fig. 1: eine Meßsonde zur Beaufschlagung der Hautoberfläche mit einem Unterdruck
- Fig. 2: eine Meßsonde zur Beaufschlagung der Hautoberfläche mit einem Überdruck und
- Fig. 3: ein Blockschaltbild der Meßvorrichtung mit Meßsonde, Druckerzeuger und Mikroprozessor.

Die Meßsonde 1 besteht im wesentlichen aus einem Anschlußstück 8 zum Anschluß einer pneumatischen Druckleitung 10 und aus einem Gehäuse 12, das einen Meßkopf 6 der Meßsonde 1 umschließt. Der Meßkopf 6 enthält eine Lichtschranke 4, bestehend aus einer als Lichtsender dienenden Infrarotleuchtdiode 14, einer als Lichtempfänger dienenden Infrarotphotodiode 16 und einem beiderseits eines Meßsondenkanals 3 angeordneten Glaskörper 18. Das Anschlußstück 8 mit einem durch das Anschlußstück verlaufenden Kanal 20 ist in den Glaskörper 18 eingesetzt, wobei der Kanal 20 in den Meßsondenkanal 3 mündet. Der Meßsondenkanal 3 weist eine Austrittsöffnung 5 auf, die bündig mit einer Auflagefläche 22 des Glaskörpers 18 abschließt. Die Dicke des Glaskörpers beträgt vorzugsweise 1 mm, wobei der Glaskörper mittig auf den Meßsondenkanal 3 gerichtet ist.

Die als Lichtsender und Lichtempfänger dienenden Infrarotdioden 14,16 verlaufen parallel zu dem Kanal 20 bzw. 3, wobei die Infrarotlichtstrahlen 24 zunächst parallel zu dem Meßsondenkanal durch eine erste geschliffene Fläche 26 des Glaskörpers 18 eintreten, die rechtwinklig zu dem Meßsondenkanal 3 verläuft. Der Glaskörper 18 weist eine unter einem Winkel von 45° zu den Infrarotlichtstrahlen 24 verlaufende erste Spiegelfläche 28 auf, die die Infrarotlichtstrahlen 24 um 90° umlenkt, so daß sie den Meßsondenkanal 3 unter einem Winkel von 90° kreuzen. Nach dem Durchstrahlen des Meßsondenkanals 3 treffen die Infrarotstrahlen 24 auf eine unter einem Winkel von 45° zu den Infrarotlichtstrahlen 24 verlaufende zweite Spiegelfläche 30, die erneut die Infrarotlichtstrahlen um 90° umlenkt und sie auf die Infrarotdiode 16 richtet, wobei sie den Glaskörper an einer quer zur Längsachse des Meßsondenkanals 3 verlaufenden zweiten geschliffenen Fläche 32 des Glaskörpers 18 verlassen. Die Spiegelflächen 28,30 reichen bis zur Auflagefläche 22 des Glaskörpers 18 und ermöglichen somit die Abtastung des Meßsondenkanals 3 bis unmittelbar in die Ebene der Auflagefläche 22. Die Spiegelflächen ermöglichen dabei die Umlenkung des vollständigen von der IR-Diode 14 ausgehenden Lichtstrahls.

Die Infrarotdioden 14,16 sind mit Anschlüssen 34,36 versehen, die mit einer elektronischen Schaltung 38 zur Steuerung des Lichtsenders und zur Verstärkung des Meßsignals des Lichtempfängers verbunden sind, die an der Meßsonde 1 befestigt ist.

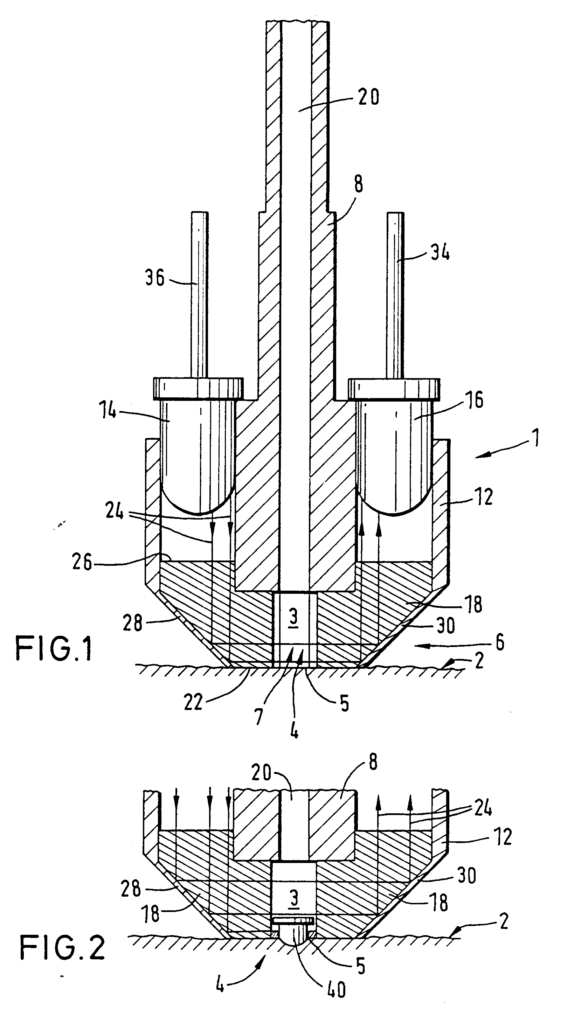

Der Kanal 20 des Anschlußstücks 8 und damit der Meßsondenkanal 3 werden über die pneumatische Druckleitung 10 in dem Ausführungsbeispiel der Fig. 1 mit einem Unterdruck beaufschlagt, wodurch die Hautoberfläche 2 an der Austrittsöffnung 5 je nach Flexibilität der Hautoberfläche unterschiedlich weit in den Meßsondenkanal 3 hineingesogen wird. Dadurch ergibt sich eine Schwächung der von dem Lichtsender auf den Lichtempfänger übertragenen Lichtintensität, die als Meßsignal für die maximale Höhe der Hautwölbung und damit für die Elastizität der Haut verwendet wird.

Fig. 2 zeigt ein anderes Ausführungsbeispiel, bei dem der Kanal 20 und der Meßsondenkanal 3 mit einem Druck beaufschlagt wird, wodurch ein bewegliches Kolbenelement 40 in die Hauptoberfläche 2 eingedrückt wird. Das druckbeaufschlagte Ende des beweglichen Kolbenelementes ragt in die Meßstrecke 7 der Lichtschranke 4 hinein und beeinflußt somit je nach Eindellung der Hautoberfläche 2 die von dem Lichtsender auf den Lichtempfänger übertragene Lichtintensität. Das auf die Hautoberfläche drückende Ende des Kolbenelementes kann geradzylindrisch, kalottenförmig oder spitz auslaufend gestaltet sein.

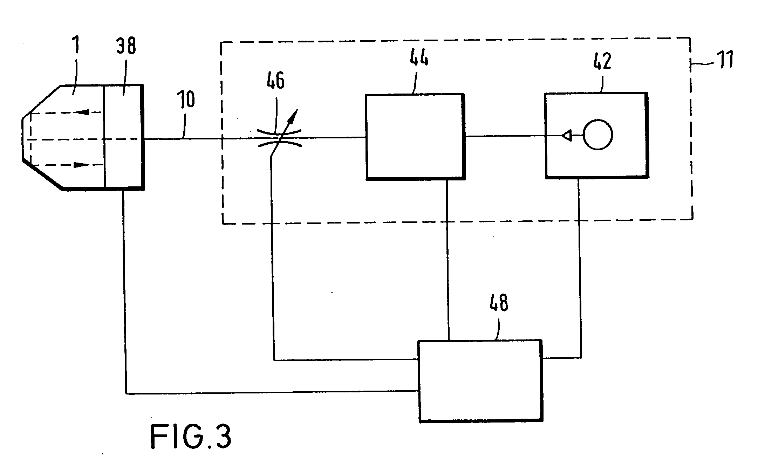

Fig. 3 zeigt ein Blockschaltbild der Vorrichtung. Der Meßkopf 1 ist über die pneumatische Druckleitung 10 aus einem Silikonschlauch mit einer Druckversorgungseinrichtung 11 verbunden, die wahlweise Überdruck oder Unterdruck zur Verfügung stellen kann. Die Druckversorgungseinrichtung 11 besteht aus einer Vakuum- bzw. Druckpumpe 42, einem Druckspeicher 44 und einem ansteuerbaren Drosselventil 46.

Der Druckspeicher 44 hat ein Fassungsvermögen von ca. 1 1, während die Pumpe 42 einen Druck von beispielsweise bis zu 500 millibar erzeugen kann.

Ein Mikroprozessor 48 steuert den Betrieb der Pumpe 42, überwacht den Druck in dem Druckspeicher 44, und steuert das Drosselventil 46 und die elektronische Schaltung 38 an dem Meßkopf 1 entsprechend einem vorgegebenen Programm an.

Mit Hilfe des Mikroprozessors ist ein bestimmter Ablauf der durchzuführenden Messungen einstellbar, wobei hinsichtlich des Druckaufbaus beispielsweise folgende Möglichkeiten bestehen:
1. Unter-/Überdruck liegt beim Aufsetzen der Meßsonde schon an.
2. Unterdruck wird von dem Umgebungsdruck auf einen vorbestimmten Sollwert linear erhöht.
3. Ein zwischen zwei Druckwerten schwankender Druck als Wechselbelastung, z.B. neunfacher Wechsel der Druckbelastung.

Hinsichtlich des Druckabbaus bei Beendigung der Messung besteht die Möglichkeit des sofortigen Druckabfalls und des allmählich auf Atmosphärendruck abfallenden Druckes.

Die Austrittsöffung 5 des Meßsondenkanals 3 kann unterschiedliche Durchmesser aufweisen. Beispielsweise ist die Messung der Hautflexibilität oberster Hautschichten in vorteilhafter Weise mit einem Querschnitt der Austrittsöffnung 5 von 2 mm durchzuführen. Sollen tiefere Hautschichten untersucht werden, ist ein Austrittsdurchmesser von ca. 8 mm vorteilhafter.

Hierzu ist vorgesehen, daß der Meßkopf 6 der Meßsonde 1 auswechselbar gestaltet ist, so daß unterschiedliche Meßköpfe mit unterschiedlichen Austrittsöffnungen oder Austrittsöffnungsformen verwendet werden können.

## Patentansprüche

1. Vorrichtung zur Messung der elastischen und viskoelastischen Verformbarkeit von Haut mit einer Meßsonde (1), die eine Wegmeßeinrichtung zur Messung des Deformationsweges der Hautoberfläche aufweist, und mit einer elektronischen Auswerteeinrichtung (38), wobei,
- die Meßsonde (1) einen die Hautoberfläche (2) mit einem pneumatischen Druck beaufschlagenden Meßsondenkanal (3) aufweist, **dadurch gekennzeichnet**
- daß zur Wegmessung im Bereich des Meßsondenkanals (3) eine Lichtschranke (4) angeordnet ist, deren Meßstrecke (7) quer zum Meßsondenkanal (3) bis zur Ebene seiner Austrittsöffnung (5) verläuft.

2. Vorrichtung nach Anspruch 1, dadurch gekennzeichnet, daß der Meßkopf (6) der Meßsonde (1) zumindest teilweise aus einem Glaskörper (18) besteht, durch den die Meßstrecke (7) verläuft.

3. Vorrichtung nach Anspruch 1 oder 2, dadurch gekennzeichnet, daß der Lichtsender und der Lichtempfänger der Lichtschranke auf gegenüberliegenden Seiten in die Meßsonde (1) integriert sind und daß die Meßstrecke (7) der Lichtschranke (4) über Spiegel (28,30) zweifach umgelenkt ist.

4. Vorrichtung nach Anspruch 1 bis 3, dadurch gekennzeichnet, daß in die Meßsonde (1) eine elektronische Schaltung (38) zur Steuerung des Lichtsenders und zur Verstärkung des Meßsignals des Lichtempfängers integriert ist.

5. Vorrichtung nach Anspruch 1 bis 4, dadurch gekennzeichnet, daß der Lichtsender aus einer Infrarotleuchtdiode (14) und der Lichtempfänger aus einer Infrarotphotodiode (16) besteht.

6. Vorrichtung nach Anspruch 5, dadurch gekennzeichnet, daß das Infrarotlicht moduliert ist.

7. Vorrichtung nach einem der Ansprüche 1 bis 6, dadurch gekennzeichnet, daß die Austrittsöffnung (5) des Meßsondenkanals (3) einen Durchmesser zwischen ca. 2 und ca. 8 mm aufweist.

8. Vorrichtung nach einem der Ansprüche 1 bis 7, dadurch gekennzeichnet, daß der Meßkopf (6) der Meßsonde (1) auswechselbar ist.

9. Vorrichtung nach einem der Ansprüche 1 bis 8, dadurch gekennzeichnet, daß die Meßsonde (1) mit einem von einer Feder vorgespannten Meßkopf (6) versehen ist.

10. Vorrichtung nach einem der Ansprüche 1 bis 9, dadurch gekennzeichnet, daß eine pneumatische Druckversorgungseinrichtung (11), bestehend aus einer Pumpe (42), einem Druckspeicher (44) und einem Drosselventil (46) den Druck im Meßsondenkanal (3) erzeugt, wobei die Steuerung der Pumpe (42), des Druckspeicherdrucks und der Drosselventilstellung über einen Mikroprozessor (48) erfolgt, der auch die elektronische Schaltung (38) der Meßsonde (1) ansteuert.

11. Vorrichtung nach Anspruch 10, dadurch gekennzeichnet, daß der Druck in dem Meßsondenkanal (3) ein Unterdruck ist.

12. Vorrichtung nach Anspruch 10, dadurch gekennzeichnet, daß der Druck in dem Meßsondenkanal (3) ein Überdruck ist und daß ein in dem Meßsondenkanal (3) frei bewegliches, mit dem Überdruck beaufschlagtes Kolbenelement (40) in die Meßstrecke (7) der Lichtschranke (4) mit seinem druckbeaufschlagten Ende hineinragt.

13. Verfahren zur Messung der elastischen und viskoelastischen Verformbarkeit von Haut durch Beaufschlagung der Hautoberfläche mit einem pneumatischen Druck über eine Meßsonde und durch Messen der Hautdeformation auf Grund der Druckbelastung über eine Deformationswegmessung, wobei
- der pneumatische Druck auf die zu messende Hautoberfläche über einen Meßsondenkanal aufgebracht wird, **dadurch gekennzeichnet,**
- daß die Hautdeformation mit Hilfe einer in dem druckbeaufschlagten Meßsondenkanal angeordneten Lichtschranke gemessen wird, deren Änderung der Lichtintensität als Maß für die Deformation der Hautoberfläche verwendet wird.

14. Verfahren nach Anspruch 13, dadurch gekennzeichnet, daß die Hautoberfläche mit einem Unterdruck beaufschlagt wird und daß die von der Photodiode gemessene Lichtintensität durch die in die Meßstrecke angesaugte Haut verringert wird.

15. Verfahren nach Anspruch 13, dadurch gekennzeichnet, daß die Hautoberfläche über einen frei im Meßsondenkanal beweglichen Kolbenelement mit einem Überdruck beaufschlagt wird und daß die Lichtintensität der Lichtschranke durch die Lage des Kolbenelementes beeinflußt wird.

16. Verfahren nach Anspruch 14 oder 15, dadurch gekennzeichnet, daß die Meßsonde mit bereits anliegendem Unter- bzw. Überdruck auf die Hautoberfläche aufgesetzt wird.

17. Verfahren nach Anspruch 14 oder 15, dadurch gekennzeichnet, daß nach dem Aufsetzen der Meßsonde der Unterdruck bzw. Überdruck von dem Umgebungsdruck auf einen Sollwert erhöht wird.

18. Verfahren nach Anspruch 14 oder 15, dadurch gekennzeichnet, daß nach dem Aufsetzen der Meßsonde eine Wechselbelastung auf die Hautoberfläche ausgeübt wird, indem der Unter- bzw. Überdruck zwischen einem ersten und einem zweiten Grenzwert mehrfach verändert wird.

## Claims

1. A device for measuring the elastic and elastico-viscous deformability of skin comprising a measuring probe (1) provided with a path-measuring device for measuring the deformation path of the skin surface, and having an electronic evaluation device (38), the measuring probe (1) having a measuring probe channel (3) through which pneumatic pressure is exerted on the skin surface (2),
characterized in that
- a light barrier (4) is disposed in the region of said measuring probe channel (3) for measuring the path, the measured length (7) of said light barrier extending transversally to said measuring probe channel (3) up to the plane of the outlet (5) thereof.

2. The device according to claim 1, characterized in that the measuring head (6) of the measuring probe (1) consists at least partly of a glass body (18) through which the measured length (7) extends.

3. The device according to claim 1 or 2, characterized in that the light emitter and the light receiver of the light barrier are integrated into said measuring probe (1) on opposite sides and that the measured length (7) of said light barrier (4) is deflected twice by mirrors (28, 30).

4. The device according to one of claims 1 to 3, characterized in that an electronic circuit (38) for controlling the light emitter and for amplifying the measuring signal of the light receiver is integrated in said measuring probe (1).

5. The device according to one of claims 1 to 4, characterized in that the light emitter consists of an infrared light diode (14) and the light receiver consists of an infrared photodiode (16).

6. The device according to claim 5, characterized in that the infrared light is modulated.

7. The device according to one of claims 1 - 6, characterized in that the outlet (5) of the measuring probe channel (3) has a diameter between approximately 2 and approximately 8 mm.

8. The device according to one of claims 1 to 7, characterized in that said measuring head (6) of said measuring probe (1) is removable.

9. The device according to one of claims 1 to 8, characterized in that said measuring probe (1) is provided with a measuring head (6) biased by a spring.

10. The device according to one of claims 1 to 9, characterized in that a pneumatic pressure supply device (11) including a pump (42), a pressure accumulator (44) and a throttle valve (46) generates the pressure in the measuring probe channel (3), the control of the pump (42), of the pressure in the pressure accumulator and of the throttle valve position being performed via a microprocessor (48) that also drives the electronic circuit (38) of the measuring probe (1).

11. The device according to claim 10, characterized in that the pressure in said measuring probe channel (3) is a vacuum.

12. The device according to claim 10, characterized in that the pressure in said measuring probe channel (3) is an overpressure and that a piston element (40) that is freely movable in said measuring probe channel (3) and on which the overpressure acts, protrudes into the measured length (7) of said light barrier (4) with the end on which the pressure acts.

13. A method for measuring the elastic and elastico-viscous deformability of skin by exerting pneumatic pressure on the skin surface via a measuring probe and by measuring the deformation of the skin caused by the pressure load by means of measuring the deformation path, the pneumatic pressure being applied via a measuring probe channel onto the skin surface to be measured,
characterized in that
- the deformation of the skin is measured by means of a light barrier disposed in the pressurized channel, the change in light intensity of said light barrier being used as a measure of the deformation of the skin surface.

14. The method according to claim 13, characterized in that the skin surface is exposed to a vacuum and that the light intensity measured by the photodiode is reduced by the skin sucked into the measured length.

15. The method according to claim 13, characterized in that the skin surface is exposed to an overpressure via a piston element disposed in a freely movable manner in said measuring probe channel and that the light intensity of said light barrier is influenced by the position of the piston element.

16. The method according to claim 14 or 15, characterized in that the measuring probe is set on the skin with the vacuum or the overpressure already present, respectively.

17. The device according to claim 14 or 15, characterized in that the vacuum or the overpressure is increased from the atmospheric pressure level to a set value after the measuring probe has been set on the skin.

18. The method according to claim 14 or 15, characterized in that, after the measuring probe has been set on the skin, a changing load is exerted on the skin surface by varying the vacuum or the overpressure between a first and a second limit value.

## Revendications

1. Dispositif pour mesurer la déformabilité élastique et visco-élastique de la peau, avec une sonde de mesure (1) présentant un dispositif de mesure de trajet, destiné à mesurer le trajet de déformation de la surface de la peau, et un dispositif d'évaluation électronique (32), la sonde de mesure (1) présentant un canal de sonde de mesure (3) admettant une pression pneumatique vers la surface de la peau (2), caractérisé en ce que, pour la mesure du trajet, il est disposé, à l'endroit du canal de sonde de mesure (3), une barrière lumineuse (4) dont le trajet de mesure (7) s'étend, transversalement au canal de sonde de mesure (3), jusqu'au plan de son orifice de sortie (5).

2. Dispositif suivant la revendication 1, caractérisé en ce que la tête de mesure (6) de la sonde de mesure (1) consiste au moins en partie en un élément en verre (18) à travers lequel s'étend le trajet de mesure (7).

3. Dispositif suivant la revendication 1 ou 2, caractérisé en ce que l'émetteur de lumière et le récepteur de lumière de la barrière lumineuse sont intégrés dans la sonde de mesure (1), sur des côtés opposés, et que le trajet de mesure (7) de la barrière lumineuse (4) est dévié deux fois par des miroirs (28, 30).

4. Dispositif suivant l'une des revendications 1 à 3, caractérisé en ce que dans la sonde de mesure (1) est intégré un circuit électronique (38) destiné à la commande de l'émetteur de lumière et à l'amplification du signal de mesure du récepteur de lumière.

5. Dispositif suivant l'une des revendications 1 à 4, caractérisé en ce que l'émetteur de lumière consiste en une diode électroluminescente à infrarouge (14) et le récepteur de lumière en une photodiode à infrarouge (16).

6. Dispositif suivant la revendication 5, caractérisé en ce que la lumière infrarouge est modulée.

7. Dispositif suivant l'une des revendications 1 à 6, caractérisé en ce que l'orifice de sortie (5) du canal de sonde de mesure (3) présente un diamètre compris entre environ 2 et environ 8 mm.

8. Dispositif suivant l'une des revendications 1 à 7, caractérisé en ce que la tête de mesure (6) de la sonde de mesure (1) est échangeable.

9. Dispositif suivant l'une des revendications 1 à 8, caractérisé en ce que la sonde de mesure (1) est pourvue d'une tête de mesure (6) prétendue par un ressort.

10. Dispositif suivant l'une des revendications 1 à 9, caractérisé en ce qu'un dispositif d'alimentation de pression pneumatique (11), composé d'une pompe (42), d'un accumulateur de pression (44) et d'une vanne d'étranglement (46), génère la pression dans le canal de sonde de mesure (3), la commande de la pompe (42), de la pression de l'accumulateur de pression et de la position de la vanne d'étranglement s'effectuant par un micro-processeur (48) qui commande également le circuit électronique (38) de la sonde de mesure (1).

11. Dispositif suivant la revendication 10, caractérisé en ce que la pression dans le canal de sonde de mesure (3) est une dépression.

12. Dispositif suivant la revendication 10, caractérisé en ce que la pression dans le canal de sonde de mesure (3) est une surpression et qu'un élément de piston (40), soumis à la surpression et pouvant se déplacer librement dans le canal de sonde de mesure (3), pénètre, par son extrémité soumise à la pression, dans le trajet de mesure (7) de la barrière lumineuse (4).

13. Procédé pour mesurer la déformabilité élastique et visco-élastique de la peau par admission, par l'intermédiaire d'une sonde de mesure, d'une pression pneumatique vers la surface de la peau et par mesure de la déformation de la peau du fait de la charge de pression par une mesure du trajet de déformation, la pression pneumatique étant appliquée, par l'intermédiaire d'un canal de sonde de mesure, sur la surface de la peau à mesurer, caractérisé en ce que la déformation de la peau est mesurée à l'aide d'une barrière lumineuse, disposée dans le canal de sonde de mesure à admission de pression, dont la modification de l'intensité lumineuse est utilisée comme mesure pour la déformation de la surface de la peau.

14. Procédé suivant la revendication 13, caractérisé en ce que la surface de la peau est soumise à une dépression et que l'intensité lumineuse mesurée par la photodiode est diminuée par la peau aspirée dans le trajet de mesure.

15. Procédé suivant la revendication 13, caractérisé en ce que la surface de la peau est soumise à une surpression, par l'intermédiaire d'un élément de piston pouvant se déplacer librement dans le canal de sonde de mesure, et que l'intensité lumineuse de la barrière lumineuse est influencée par la position de l'élément de piston.

16. Procédé suivant la revendication 14 ou 15, caractérisé en ce que la sonde de mesure est placée sur la surface de la peau avec la dépression ou surpression déjà présente.

17. Procédé suivant la revendication 14 ou 15, caractérisé en ce qu'après la pose de la sonde de mesure, la dépression ou surpression est augmentée, de la pression ambiante, à une valeur de consigne.

18. Procédé suivant la revendication 14 ou 15, caractérisé en ce qu'après la pose de la sonde de mesure, il est exercé une sollicitation alternée sur la surface de la peau en modifiant plusieurs fois la dépression ou la surpression entre une première et une seconde valeur limite.
